# EUROPEAN PATENT SPECIFICATION

(11) **EP 3 296 385 B1**
(45) Date of publication and mention of the grant of the patent: **09.10.2019**
(21) Application number: 15732013.6
(22) Date of filing: 13.05.2015
(51) Int. Cl.: C12M 1/00, C12M 1/12, C12M 3/00

(54) **MODULAR PHOTOBIOREACTORS SYSTEM FOR THE CULTIVATION OF ALGAE**
MODULARES SYSTEM MIT PHOTOBIOREAKTOREN ZUM ZÜCHTEN VON ALGEN
SYSTÈME MODULAIRE DE PHOTOBIORÉACTEURS POUR LA CULTUREM D'ALGUES

(43) Date of publication of application: 21.03.2018
(73) Proprietor: AAA Accelerator Group Europe AG, 6317 Oberwil B. Zug (CH)
(72) Inventor: Mahoney, Emilio Alexander, 28005 Madrid (ES); Álvarez Garcia, Luis, 28005 Madrid (ES); Bacchetta, Tino Luigi, 28005 Madrid (ES); Bacchetta, Renzo Christopher, 28005 Madrid (ES)
(74) Representative: Wallinger Ricker Schlotter Tostmann
(86) International application number: PCT/ES2015/070382
(87) International publication number: WO 2016/180995

(56) References cited:
- WO-A1-2012/058756
- WO-A1-2014/130362
- ES-B1- 2 446 640
- US-A1- 2012 021 496

## Description

### OBJECT OF THE INVENTION

The present invention relates to a modular system of photobioreactors designed for algae farming.

The object of the invention is a modular system of closed photobioreactors, intended for indoor operation with LED lighting, uninterrupted and completely automated, with continuous monitoring of process variables, and feedback control of CO2 and nutrients, of water temperature along the entire route of the photobioreactor, allowing maximum use of indoor space where the system is arranged.

### BACKGROUND OF THE INVENTION

The continued production of algal oil and biomass requires three distinct phases: algae production, product collection and processing for generation of oil and biomass for use, in order to produce biodiesel, cosmetics, pharmaceuticals and other products.

Today algae farming is done mainly in outdoor ponds and in photobioreactors made of plastic or glass that may have tubular configurations with diameters ranging mostly from 10 cm to 30 cm.

These systems are generally installed outdoors and operate by pumping water and algae, through a circuit in which carbon dioxide is injected into the tank through which it is pumped. With the action of sunlight algae grow along said circuit. However, these systems have no control over the light spectrums to which algae are subjected, nor on the amount of nutrients, or carbon dioxide required and nor do they control or determine the temperature of the water which the cultivation is grown.

For greater control over light spectra and for proper temperature control, alternatively the photobioreactor can be located indoors where artificial light is applied in the absence of the infrared and ultraviolet spectra which are harmful to algae, and where at least the temperature of the room in which the photobioreactor is located is controlled.

Furthermore and in order to protect the algae, the use of photobioreactors configured by a closed circuit is known, so that, unlike open and external systems, the algae are protected against microorganisms and other harmful agents that have negative effects on algae growth and prevent algae infestation.

The above include the Spanish Patent ES2446640B1 of the same applicant which discloses a photobioreactor for growing algae for indoor use, comprising LED-type lights, and an airtight sealed tubular conduit on which the lights are placed, formed by several tubular sections facing each other and connected together at their ends.

Said photobioreactor further comprises supply nozzles located at the base of the tubular sections, one CO₂ supply line, a nutrient supply line and various temperature conditioned water supply lines, running near and along the route of the tubular conduit, which are connected to said supply nozzles and which are equipped with flow valves

This photobioreactor further comprises a plurality of sensors distributed evenly by the tubular conduit to detect the need to incorporate nutrients, of CO₂ and to increase or decrease the water temperature, and a control unit, receiving the signals from the sensors, and which automatically operates the flow valves of the supply lines for supplying an additional amount of CO₂, nutrients or temperature conditioned water to the various pipe sections, depending on the needs of the cultivation process in view of the signals detected by the sensors and a treatment plant located at the end of the photobioreactor for the final treatment of the product.

Due to the large size of the said photobioreactor, it is necessary to have large indoor spaces to carry out their installation, plus hang elements that support the LED lighting, which makes the use of the indoor space very reduced, with associated increased costs and energy consumption.

The present invention overcomes all the above drawbacks through a modular photobioreactor system that allows maximum use of the indoor space where it is arranged, and a very low power consumption

### DESCRIPTION OF THE INVENTION

The modular system of photobioreactors for algae farming of the present invention comprises at least two closed tubular-type photobioreactors, designed to operate continuously in an indoor location, in which storage tanks are located at the front of the modular system and product processing circuits are located at the end of the modular system to produce algae oil and biomass, wherein at least two photobioreactors can be coupled next to each other and / or at different levels.

The modular system of photobioreactors object of this invention is entirely automated to control the growing process in each of the sections of each of the photobioreactor, by data collection and feedback in each of these sections, of the elements and characteristic parameters of the process of cultivating algae, preferably the amount of nutrients, amount of carbon dioxide required, and the water temperature in which the cultivation is grown.

An object of the present invention is therefore the particular arrangement of the modular system of photobioreactors and complementary installations to facilitate measurement and supply of nutrients, carbon dioxide and temperature conditioned water along the sections which define each photobioreactor, as well as the regulation of the above parameters by a control unit.

Each photobioreactor of the modular system of photobioreactors of the present invention comprises a metallic structure which in turn it comprises at least two tubular sections connected by one of its ends, wherein on the inside of said tubular sections the algae culture is arranged and wherein between said tubular sections there is another tubular section comprising LED-type lights and reflector bodies that reflect the light of the LED type lighting and transmit it to the tubular sections in which the cultivation of algae is disposed passing through their side walls.

Moreover, it is noteworthy that the tubular sections of each bioreactor in which the algae cultivation is arranged have a section of trapezoidal configuration, with their lateral sides having a downwardly converging inclination, wherein said sides are made of thermal glass to maximally reduce the heat loss of the water in the interior of each photobioreactor. Each bioreactor is divided into several tubular sections, which can be about 12 meters, and each one of said sections has at its base supply nozzles conveniently distributed that lead to the inlet of CO₂ or water or nutrients, depending on the supply line that is connected. Therefore, the tubular sections of each bioreactor where the LED-type lights and reflector bodies are arranged, have a trapezoidal configuration section, with its lateral sides having an upwardly convergent inclination.

Each photobioreactor therefore has LED-type lights that eliminate the harmful action of infrared and ultraviolet light on algae. These lights provide a spectrum of 400 nm to 700 nm so as to allow optimum and uninterrupted photosynthesis and produce algae every hour.

It is planned that the modular system of photobioreactors also will include in the control unit, means that regulate the activation of the lights, preferably controlling two types of LED, (red and blue), with 2 different spectra, so that it regulates which spectrum should be active at all times and in the most appropriate sections of the photobioreactor, to promote the growth of algae according to their colour.

This modular system of photobioreactors is preferably located in an enclosed building normally kept at room temperature between 15 and 20 °C permanently. In any case the modular system of photobioreactors itself incorporates a control system ensuring at all times optimal water temperature along all sections of each photobioreactor.

The modular system of photobioreactors is connected to a main tank or incubator which stores dechlorinated water, algae and nutrients, mixed with carbon dioxide, which is introduced into each of the bioreactors such that the resulting chemical composition provides a maximum growth of algae.

For this reason provided along the circuit of each of the photobioreactors a series of sensors are arranged that measure the characteristic parameters of the process, for example located every 6 linear meters along the photobioreactor, and which transmit to the control unit, which in a fully automated manner injects carbon dioxide and / or nutrients and / or hot or cold water, depending on the rate of growth of algae in different sections of each photobioreactor, which determines the rate of optimal growth, obtaining thus a higher production than other systems.

The modular system of photobioreactors also has a self-cleaning system by scanning injectors allowing uninterrupted operation which is not possible in other closed circuit systems.

The whole process, from the introduction of the algae into modular system of photobioreactors to the production of biodiesel is done in a fully automated manner.

The size and configuration of the section of the tubular sections, in combination with the location of the supply nozzles at the base of each section, allows for convenient movement of algae resulting in optimum production, wherein the proportion of water / air inside the tubular sections is 70/30 by volume.

Each photobioreactor includes a tubular section with a larger output than the rest of tubular sections, which forms a storage and separation tank for separating oily algae flow having the appropriate size are then directed to the treatment plant, while the rest of the flow is directed back into the main tank.

Furthermore, at the upper part of the tubular sections of each photobioreactor, the part occupied by air, they are exhaust valves to facilitate the outlet of excess oxygen and / or pressure. These valves can be located at the junction between the tubular sections every 12 m.

### DESCRIPTION OF THE DRAWINGS

To complement the description being made and in order to aid a better understanding of the characteristics of the invention according to a preferred practical embodiment thereof, attached as an integral part of said description, is a set of drawings wherein by way of illustration without limiting the scope of the invention, the following has been represented:
Figure 1 shows an elevational view of the modular system of photobioreactors of the present invention in which the arrangement of the tubular sections of the photobioreactors is observed, and the structure that surrounds them and supports both the LED-type lights and the reflectors.
Figure 2 shows a sectional view AA of Figure 1, wherein the storage tanks are also included at the front of the photobioreactors and the product treatment systems located at the end of the photobioreactors.
Figure 3 shows a side view of Figure 2.
Figure 4 shows a view of the detail B of Figure 3, wherein the part of the structure separating a first photobioreactor and a second photobioreactor disposed above the first photobioreactor are shown.
Figure 5 shows an exploded view of the location of the sensors in a section of each of the photobioreactors.

### PREFERRED EMBODIMENT OF THE INVENTION

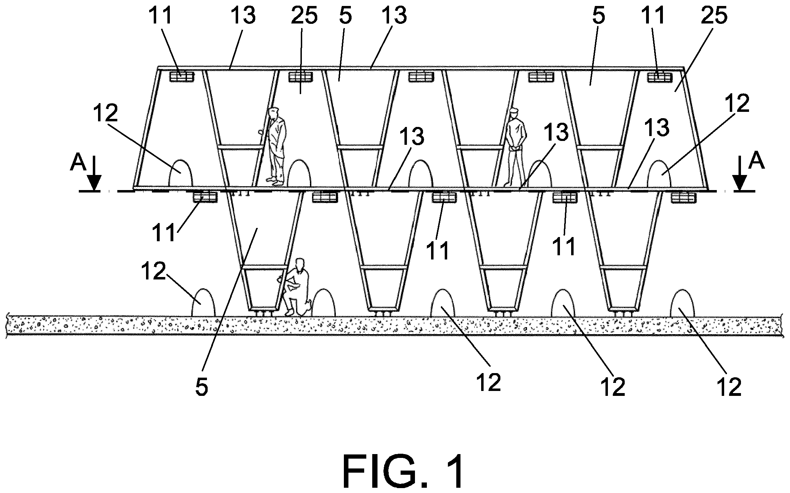

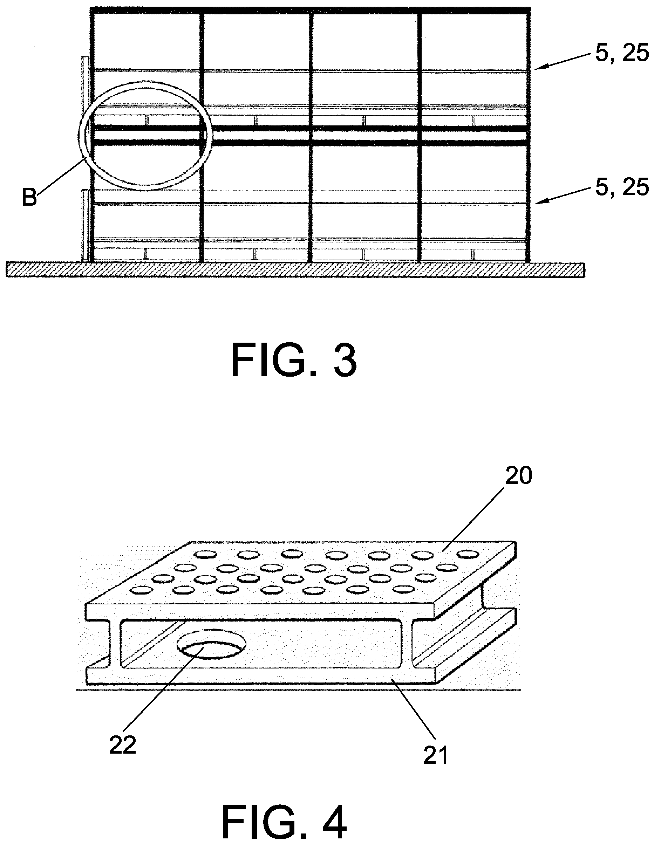

Described below is a preferred embodiment of the invention, with reference to FIGS 1 to 5 described above.

Figure 1 shows an elevational a view of a first embodiment of the modular system of photobioreactors of the present invention formed by a first photobioreactor and a second photobioreactor disposed above the first photobioreactor, wherein each photobioreactor comprises a metal structure in turn comprising at least two tubular sections (5) connected by one of their ends, wherein the inside of said tubular sections (5) the algae cultivation is arranged and wherein between said tubular sections (5) another tubular section (25) is arranged comprising LED-type lights (11) and reflector bodies (12) reflecting light from the LED-type lights (11) and transmitting it toward the tubular sections (5) wherein the algae cultivation is arranged, passing through their side walls.

The tubular sections (5) of each bioreactor wherein the algae cultivation is arranged have a section of trapezoidal configuration, with their lateral sides having a downwardly converging inclination to receive light from said reflector bodies (12). These lateral sides are preferably made of thermal glass for better use of light. It is also possible that each of the tubular sections (5) has at least a folding top cover (13) relative to a lateral side permitting access therein. Therefore the tubular sections (25) of each bioreactor in which the LED-type lights (11) and the reflector bodies (12) are arranged, have a section of trapezoidal configuration, with its lateral sides having an upwardly convergent inclination.

In this first embodiment, the modular system of photobioreactors further comprises a drainage structure disposed between the metal structure of the first photobioreactor and the metal structure of the second photobioreactor, wherein said drainage structure comprises a perforated top wall (20) and a lower wall (21) with a drainage hole (22) so that the water flows through the perforated top wall (20) to the drainage hole (22) in case of water spilling from the second photobioreactor disposed above the first photobioreactor.

In a second embodiment, the photobioreactors are arranged side by side.

In a third embodiment, a combination of the first and second embodiment, the system comprises at least three photobioreactors which include a first photobioreactor, a second and a third photobioreactor photobioreactor, wherein the third bioreactor is arranged beside the first bioreactor and wherein the second bioreactor is disposed above or below the first or third photobioreactor.

The modular system of photobioreactors is arranged within a facility in which there is the main tank or incubator (1) containing dechlorinated water, algae and nutrient, saturated with a mixture of CO₂, CO₂ tanks (2) and a nutrient tanks (3), in correspondence with the inlet of each photobioreactor and a treatment plant (4) located at the end of the photobioreactor for the final treatment of the product.

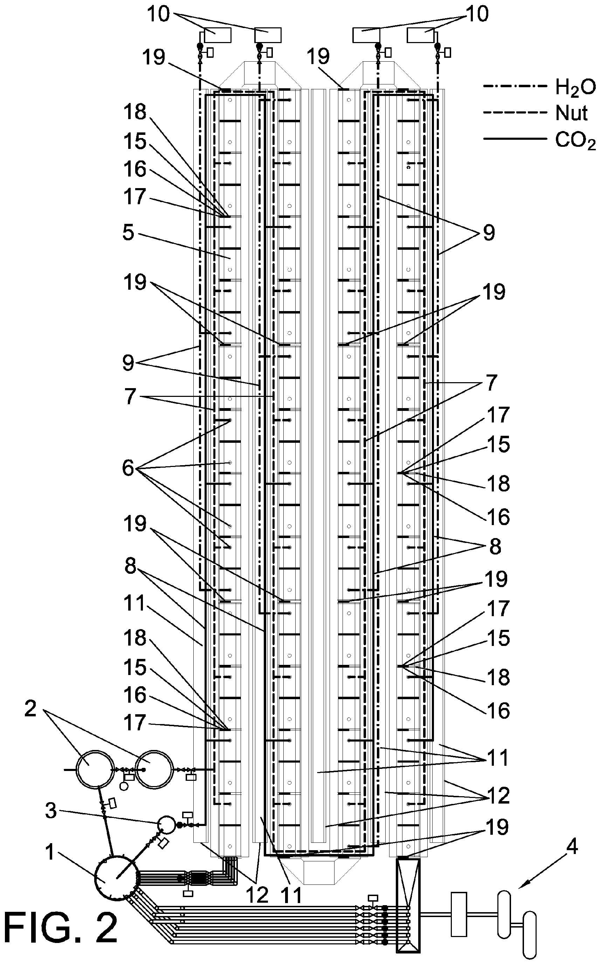

The tubular sections (5) wherein the algae cultivation is arranged include at their base at least one supply nozzle (6), and comprising a CO₂ supply line (7), a nutrient supply line (8) and several temperature conditioned water supply lines (9), which are distributed near and along the tubular conduit, which are connected to the said supply nozzles (6), as seen in greater detail in Figure 2 and that are equipped with flow valves.

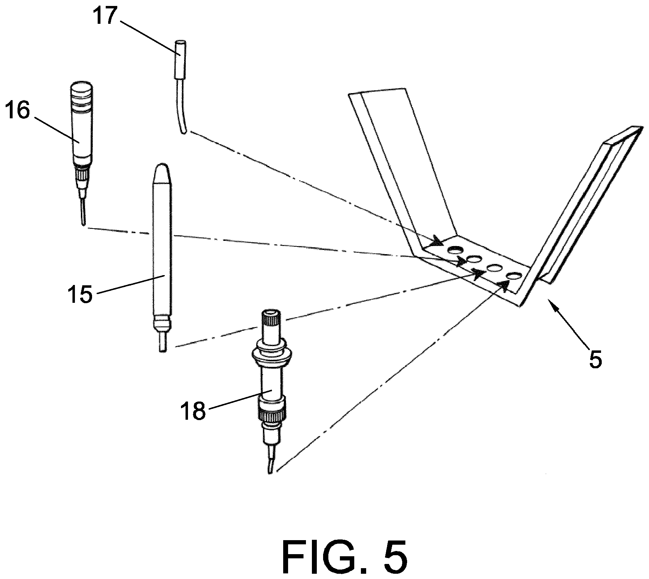

Also, each photobioreactor comprises evenly distributed tubular sections (5) wherein the algae cultivation is arranged, and a series of nutrient sensors (15) detecting the need to incorporate nutrients, CO₂ sensors (16), temperature sensors (17) which detect the need to increase or decrease the water temperature and pH sensors (18), all shown in Figure 5, and has a control unit, receiving the signals from the sensors, and automatically acting on the flow valves of the supply lines (7, 8, 9) for supplying an additional amount of CO₂, nutrients or temperature conditioned water to different tubular sections (5), depending on the process needs in view of the signals detected by the sensors.

As seen in Figure 2, the supply line of CO₂ (7) is connected and feeds the CO₂ tank (2) and the supply line of nutrients (8) is connected and feeds the nutrient tank (3).

Furthermore, each of the temperature conditioned water supply lines (9) extend in correspondence with different sectors of each bioreactor. Here they are distributed in correspondence with each parallel longitudinal sector of the bioreactor, as shown in Figure 2, to establish an independent temperature regulation of each of said sectors each of said temperature conditioned water supply lines (9) being connected to a separate heating unit (10), normally located, as shown in Figure 2, at the end of each sector positioned opposite the location of the photobioreactor inlet tubular section.

The initial tubular section (5) of each photobioreactor, which occupies the inlet position and which is reached by the product that houses the main tank (1) has a series of openings (14) evenly distributed on the front face, here being 6 in number, designed to receive as many conduits that carry product from the main tank (1) to each photobioreactor, thus facilitating uniform inlet and distribution of the product flow from the main tank (1) in each photobioreactor.

## Claims

1. Modular system of photobioreactors for algae cultivation comprising at least two photobioreactors each of which comprises a metallic structure in turn comprising at least two tubular sections (5) connected by one of its ends, wherein inside said tubular sections (5) the algae cultivation is arranged and wherein between said tubular sections (5), the system comprises LED-type lights (11) and reflector bodies (12) that reflect the light from the LED-type lighting (11) and transmit it to the tubular sections (5) wherein the cultivation of algae is disposed, passing through their side walls **characterized in that** the system further comprises another tubular section (25) disposed between said tubular sections (5), wherein the other tubular section (25) comprises the LED-type lights (11) and the reflector bodies (12).

2. Modular system of photobioreactors for algae cultivation according to claim 1 **characterized in that** the tubular sections (5) of each bioreactor wherein the algae cultivation is arranged have a section of trapezoidal configuration, with its lateral sides having a downwardly convergent inclination to receive light from the reflector bodies (12) and the tubular sections (25) of each bioreactor wherein the LED-type lights are arranged (11) and the reflector bodies (12) have a section of trapezoidal configuration, with its lateral sides having an upwardly convergent inclination.

3. Modular system of photobioreactors for algae cultivation according to either claim 1 or 2 **characterized in that** it comprises at least two photobioreactors including a first photobioreactor and a second photobioreactor disposed above the first photobioreactor.

4. Modular system of photobioreactors for algae cultivation according to either claim 1 or 2 **characterized in that** it comprises at least two photobioreactors including a first photobioreactor and a second photobioreactor disposed beside the first photobioreactor.

5. Modular system of photobioreactors for algae cultivation according to either claim 1 or 2 **characterized in that** it comprises at least three photobioreactors including a first photobioreactor, a second photobioreactor and a third photobioreactor, wherein the third photobioreactor is disposed next to the first bioreactor and wherein the second bioreactor is disposed above or below the first or third photobioreactor.

6. Modular system of photobioreactors for algae cultivation according to either claim 3 or 5 **characterized by** further comprising a drainage structure disposed between the metal structure of the first photobioreactor and the metal structure of the second photobioreactor, wherein said drainage structure comprises a perforated top wall (20) and a lower wall (21) with a drainage hole (22) so that the water flows through the perforated top wall (20) to the drainage hole (22) in case of spillage of water from the second bioreactor disposed above the first photobioreactor.

7. Modular system of photobioreactors for algae cultivation according to any of the preceding claims **characterized by** further comprising:
supply nozzles (6) located at the base of the tubular sections (5) wherein the algae cultivation is arranged,
a CO₂ supply line (7), a nutrient supply line (8) and several temperature conditioned water supply lines (9), which are distributed near and along the route of the tubular sections (5) wherein the growing algae is disposed, which are connected to said supply nozzles (6), and are equipped with flow valves.
a series of nutrient sensors (15) detecting the need to incorporate nutrients, CO₂ sensors (16) and temperature sensors (17) detecting the need to raise or lower the water temperature, and
a control unit, which receives the signals from the sensors and which automatically operates the flow valves of the supply lines (7, 8, 9) for supplying an additional amount of CO₂, nutrients or temperature conditioned water to different tubular sections (5) in which the algae cultivation is arranged, according to the needs of the cultivation process in view of the signals detected by the sensors.

8. Modular system of photobioreactors for algae cultivation according to claim 7 **characterized in that** further comprises a series of pH sensors (18).

9. Modular system of photobioreactors for algae cultivation according to claim 7 **characterized in that** the temperature conditioned water supply lines (9) extend separately in correspondence with the tubular sections (5) in which the algae cultivation is arranged in each photobioreactor.

10. Modular system of photobioreactors for algae cultivation according to claim 9 **characterized in that** each of the temperature conditioned water supply lines (9) is connected to a heating unit (10).

11. Modular system of photobioreactors for algae cultivation according to any of the preceding claims **characterized in that** each of the tubular sections (5) in which the algae cultivation of each photobioreactor is arranged, has at least a folding top cover (13) relative to a lateral side that allows access to its interior.

12. Modular system of photobioreactors for algae cultivation according to any of the preceding claims **characterized in that** each photobioreactor has an initial tubular section (5) wherein the algae cultivation is arranged equipped on its front with face a series of evenly distributed openings (14), intended to receive as many conduits carrying the product being introduced into the photobioreactor.

13. Modular system of photobioreactors for algae cultivation according to any of the preceding claims **characterized in that** each LED-type light (11) comprises two types of LED, red and blue, with two different spectra, and the control unit has means regulating the activation of these lights (11) of two types of LEDs in different tubular sections (5) in which the cultivation of algae is arranged.

14. Modular system of photobioreactors for algae cultivation according to any of the preceding claims **characterized in that** each photobioreactor incorporates a output tubular section (5) in which the algae cultivation is arranged, being larger than the other tubular sections (5) in which the algae cultivation is arranged, which constitutes a storage and separation tank with upper outlets for separating the flow of oily algae, and with several lower outlets, with a greater flow area than the upper the outlets.

15. Modular system of photobioreactors for algae cultivation according to any of the preceding claims **characterized in that** each photobioreactor comprises in its upper part evenly distributed exhaust valves to facilitate the exit of excess oxygen and / or pressure.

## Patentansprüche

1. Modulares System von Photobioreaktoren zur Kultivierung von Algen, welches mindestens zwei Photobioreaktoren aufweist, von denen jeder eine metallische Struktur aufweist, die wiederum mindestens zwei rohrförmige Abschnitte (5) aufweist, die durch eines ihrer Enden verbunden sind, wobei innerhalb der rohrförmigen Abschnitte (5) die Kultivierung der Algen angeordnet ist und wobei das System zwischen den röhrenförmigen Abschnitten (5) Lichtquellen (11) vom Typ LED und Reflektorkörper (12) aufweist, die das Licht der Lichtquellen (11) vom Typ LED reflektieren und auf die röhrenförmigen Abschnitte (5) übertragen, in denen die Kultivierung der Algen angeordnet ist, und die durch ihre Seitenwände verlaufen, **dadurch gekennzeichnet, dass** das System ferner einen anderen rohrförmigen Abschnitt (25) aufweist, der zwischen den rohrförmigen Abschnitten (5) angeordnet ist, wobei der andere rohrförmige Abschnitt (25) die Lichtquellen (11) vom Typ LED und die Reflektorkörper (12) aufweist.

2. Modulares System von Photobioreaktoren zur Kultivierung von Algen nach Anspruch 1, **dadurch gekennzeichnet, dass** die röhrenförmigen Abschnitte (5) jedes Bioreaktors, in denen die Kultivierung der Algen angeordnet ist, einen trapezförmig ausgebildeten Abschnitt aufweisen, dessen laterale Seiten eine nach unten konvergierende Neigung aufweisen, um Licht von den Reflektorkörpern (12) und den rohrförmigen Abschnitten (25) jedes Bioreaktors zu empfangen, in denen die Lichtquellen (11) vom Typ LED angeordnet sind, und dass die Reflektorkörper (12) einen trapezförmig ausgebildeten Abschnitt aufweisen, dessen laterale Seiten eine nach oben konvergierende Neigung aufweisen.

3. Modulares System von Photobioreaktoren zur Kultivierung von Algen nach Anspruch 1 oder 2, **dadurch gekennzeichnet, dass** es mindestens zwei Photobioreaktoren aufweist, die einen ersten Photobioreaktor und einen zweiten Photobioreaktor aufweisen, der über dem ersten Photobioreaktor angeordnet sind.

4. Modulares System von Photobioreaktoren zur Kultivierung von Algen nach Anspruch 1 oder 2, **dadurch gekennzeichnet, dass** es mindestens zwei Photobioreaktoren aufweist, die einen ersten Photobioreaktor und einen zweiten Photobioreaktor aufweisen, die neben dem ersten Photobioreaktor angeordnet sind.

5. Modulares System von Photobioreaktoren zur Kultivierung von Algen nach Anspruch 1 oder 2, **dadurch gekennzeichnet, dass** es mindestens drei Photobioreaktoren aufweist, die einen ersten Photobioreaktor, einen zweiten Photobioreaktor und einen dritten Photobioreaktor aufweisen, wobei der dritte Photobioreaktor neben dem ersten Bioreaktor angeordnet ist und wobei der zweite Bioreaktor oberhalb oder unterhalb des ersten oder des dritten Photobioreaktors angeordnet ist.

6. Modulares System von Photobioreaktoren zur Kultivierung von Algen nach Anspruch 3 oder 5, **dadurch gekennzeichnet, dass** es ferner eine Drainagestruktur aufweist, die zwischen der Metallstruktur des ersten Photobioreaktors und der Metallstruktur des zweiten Photobioreaktors angeordnet ist, wobei die Drainagestruktur eine perforierte obere Wand (20) und eine untere Wand (21) mit einem Abflussloch (22) aufweist, so dass das Wasser durch die perforierte obere Wand (20) zum Abflussloch (22) fließt, wenn das Wasser aus dem zweiten Photobioreaktor verschüttet wird, die über dem ersten Bioreaktor angeordnet ist.

7. Modulares System von Photobioreaktoren zur Kultivierung von Algen nach einem der vorhergehenden Ansprüche, **gekennzeichnet durch**:
Zufuhrdüsen (6), die sich am Boden der rohrförmigen Abschnitte (5) befinden, in denen die Kultivierung der Algen angeordnet ist,
eine C0₂-Zuleitung (7), eine Nährstoffzuleitung (8) und mehrere temperierte Wasserzuleitungen (9), die in der Nähe und entlang des Verlaufs der rohrförmigen Abschnitte (5) verteilt sind, in denen sich die wachsenden Algen befinden und die mit den Zufuhrdüsen (6) verbunden sind, und die mit Durchflussventilen ausgestattet sind,
eine Reihe von Nährstoffsensoren (15), die die Notwendigkeit des Einbringens von Nährstoffen erfassen, C0₂-Sensoren (16) und Temperatursensoren (17), die die Notwendigkeit eines Erhöhens oder eines Verringerns der Wassertemperatur erfassen, und
eine Steuereinheit, die die Signale von den Sensoren empfängt und die automatisch die Durchflussventile der Zufuhrleitungen (7, 8, 9) betätigt, um eine zusätzlichen Menge an CO₂, an Nährstoffen oder an temperierten Wasser an verschiedene Rohrabschnitte (5), bei denen der Kultivierung der Algen angeordnet ist, nach den Erfordernissen des Kultivierungsprozesses im Hinblick auf die von den Sensoren erfassten Signale zuzuführen.

8. Modulares System von Photobioreaktoren zur Kultivierung von Algen nach Anspruch 7, **dadurch gekennzeichnet, dass** es ferner eine Reihe von pH-Sensoren (18) aufweist.

9. Modulares System von Photobioreaktoren zur Kultivierung von Algen nach Anspruch 7, **dadurch gekennzeichnet, dass** die Zuleitungen (9) für temperiertes Wasser in Übereinstimmung mit den Rohrabschnitten (5), in denen die Kultivierung der Algen in jedem Photobioreaktor angeordnet ist, getrennt verlaufen.

10. Modulares System von Photobioreaktoren zur Kultivierung von Algen nach Anspruch 9, **dadurch gekennzeichnet, dass** jede der temperierten Wasserzuleitungen (9) mit einer Heizeinheit (10) verbunden ist.

11. Modulares System von Photobioreaktoren zur Kultivierung von Algen nach einem der vorhergehenden Ansprüche, **dadurch gekennzeichnet, dass** jeder der rohrförmigen Abschnitte (5), in denen die Kultivierung der Algen eines jeden Photobioreaktors angeordnet ist, mindestens eine faltende obere Abdeckung (13) in Bezug auf eine seitliche Seite aufweist, die den Zugang zu seinem Inneren ermöglicht.

12. Modulares System von Photobioreaktoren zur Kultivierung von Algen nach einem der vorhergehenden Ansprüche, **dadurch gekennzeichnet, dass** jeder Photobioreaktor einen röhrenförmigen Anfangsabschnitt (5) aufweist, in dem die Kultivierung der Algen an seiner Vorderseite mit einer Reihe von gleichmäßig verteilten Öffnungen (14) versehen ist, die vorgesehen sind, um so viele Leitungen zu halten, die das Produkt tragen, das in den Photobioreaktor eingeführt wird.

13. Modulares System von Photobioreaktoren zur Kultivierung von Algen nach einem der vorhergehenden Ansprüche, **dadurch gekennzeichnet, dass** jede Lichtquelle (11) vom Typ LED zwei Arten von LEDs, rot und blau, mit zwei unterschiedlichen Spektren aufweist und dass die Steuereinheit Mittel zur Regelung der Aktivierung von diesen Lichtquellen (11) der zwei Arten vom Typ LED in den unterschiedlichen rohrförmigen Abschnitten (5) aufweist, in denen die Kultivierung der Algen angeordnet ist.

14. Modulares System von Photobioreaktoren für die Kultivierung von Algen nach einem der vorhergehenden Ansprüche, **dadurch gekennzeichnet, dass** jeder Photobioreaktor einen Ausgangsrohrabschnitt (5) enthält, in dem die Kultivierung der Algen angeordnet ist, der größer als die anderen Rohrabschnitte (5) ist, in denen die Kultivierung der Algen angeordnet ist, der einen Speicher- und Abscheidebehälter mit oberen Auslässen zur Abscheidung des Ölalgenstroms und mit mehreren unteren Auslässen mit einer größeren Strömungsfläche als die der oberen Auslässe bildet.

15. Modulares System von Photobioreaktoren zur Kultivierung von Algen nach einem der vorhergehenden Ansprüche, **dadurch gekennzeichnet, dass** jeder Photobioreaktor in seinem oberen Teil gleichmäßig verteilte Auslassventile aufweist, um den Austritt von überschüssigem Sauerstoff und / oder Druck zu erleichtern.

## Revendications

1. Système modulaire de photobioréacteurs pour la culture d'algues comprenant au moins deux photobioréacteurs dont chacun comprend une structure métallique comprenant quant à elle au moins deux sections tubulaires (5) raccordées par l'une de ses extrémités, dans lequel est agencée la culture d'algues à l'intérieur desdites sections tubulaires (5) et dans lequel entre lesdites sections tubulaires (5), le système comprend des lumières de type DEL (11) et des corps réflecteurs (12) qui réfléchissent la lumière provenant de l'éclairage de type DEL (11) et la transmettent aux sections tubulaires (5) dans lesquelles la culture d'algues est disposée, passant à travers leurs parois de côté **caractérisé en ce que** le système comprend en outre une autre section tubulaire (25) disposée entre lesdites sections tubulaires (5), dans lequel l'autre section tubulaire (25) comprend les lumières de type DEL (11) et les corps réflecteurs (12).

2. Système modulaire de photobioréacteurs pour la culture d'algues selon la revendication 1, **caractérisé en ce que** les sections tubulaires (5) de chaque bioréacteur dans lequel la culture d'algues est agencée ont une section de configuration trapézoïdale, avec ses côtés latéraux ayant une inclinaison convergente vers le bas pour recevoir la lumière des corps réflecteurs (12) et les sections tubulaires (25) de chaque bioréacteur dans lequel les lumières de type DEL (11) sont agencées et les corps réflecteurs (12) ont une section de configuration trapézoïdale, avec ses côtés latéraux ayant une inclinaison convergente vers le haut.

3. Système modulaire de photobioréacteurs pour la culture d'algues selon l'une ou l'autre des revendications 1 ou 2, **caractérisé en ce qu'**il comprend au moins deux photobioréacteurs incluant un premier photobioréacteur et un deuxième photobioréacteur disposés au-dessus du premier photobioréacteur.

4. Système modulaire de photobioréacteurs pour la culture d'algues selon l'une ou l'autre des revendications 1 ou 2, **caractérisé en ce qu'**il comprend au moins deux photobioréacteurs incluant un premier photobioréacteur et un deuxième photobioréacteur disposé au-dessus du premier photobioréacteur.

5. Système modulaire de photobioréacteurs pour la culture d'algues selon l'une ou l'autre des revendications 1 ou 2, **caractérisé en ce qu'**il comprend au moins trois photobioréacteurs incluant un premier photobioréacteur, un deuxième photobioréacteur et un troisième photobioréacteur, dans lequel le troisième photobioréacteur est disposé à côté du premier bioréacteur et dans lequel le deuxième bioréacteur est disposé au-dessus ou en dessous du premier ou troisième photobioréacteur.

6. Système modulaire de photobioréacteurs pour la culture d'algues selon l'une ou l'autre des revendications 3 ou 5, **caractérisé en ce qu'**il comprend en outre une structure de drainage disposée entre la structure de métal du premier photobioréacteur et la structure de métal du deuxième photobioréacteur, dans lequel ladite structure de drainage comprend une paroi haute perforée (20) et une paroi inférieure (21) avec un trou de drainage (22) de sorte que l'eau s'écoule à travers la paroi haute perforée (20) vers le trou de drainage (22) en cas de déversement d'eau depuis le deuxième bioréacteur disposé au-dessus du premier photobioréacteur.

7. Système modulaire de photobioréacteurs pour la culture d'algues selon l'une quelconque des revendications précédentes, **caractérisé en ce qu'**il comprend en outre :
des buses d'alimentation (6) situées au niveau de la base des sections tubulaires (5) dans lesquelles la culture d'algues est agencée,
une ligne d'alimentation en CO₂ (7), une ligne d'alimentation en nutriment (8) et plusieurs lignes d'alimentation en eau à température contrôlée (9), qui sont réparties près et le long du trajet des sections tubulaires (5) dans lesquelles les algues en cours de croissance sont disposées, qui sont raccordées auxdites buses d'alimentation (6), et sont équipées de vannes d'écoulement,
une série de capteurs de nutriment (15) détectant le besoin d'incorporer des nutriments, des capteurs de CO₂ (16) et des capteurs de température (17) détectant le besoin de monter ou baisser la température de l'eau, et
une unité de commande, qui reçoit les signaux des capteurs et qui exploite automatiquement les vannes d'écoulement des lignes d'alimentation (7, 8, 9) en vue de fournir une quantité additionnelle de CO₂, de nutriments ou d'eau à température contrôlée à des sections tubulaires différentes (5) dans lesquelles la culture d'algues est agencée, selon les besoins du processus de culture au vu des signaux détectés par les capteurs.

8. Système modulaire de photobioréacteurs pour la culture d'algues selon la revendication 7, **caractérisé en ce qu'**il comprend en outre une série de capteurs de pH (18).

9. Système modulaire de photobioréacteurs pour la culture d'algues selon la revendication 7, **caractérisé en ce que** les lignes d'alimentation en eau à température contrôlée (9) s'étendent séparément en correspondance avec les sections tubulaires (5) dans lesquelles la culture d'algues est agencée dans chaque photobioréacteur.

10. Système modulaire de photobioréacteurs pour la culture d'algues selon la revendication 9, **caractérisé en ce que** chacune des lignes d'alimentation en eau à température contrôlée (9) est raccordée à une unité chauffante (10).

11. Système modulaire de photobioréacteurs pour la culture d'algues selon l'une quelconque des revendications précédentes, **caractérisé en ce que** chacune des sections tubulaires (5) dans lesquelles la culture d'algues de chaque photobioréacteur est agencée, a au moins un couvercle haut pliant (13) par rapport à un côté latéral qui permet l'accès à son intérieur.

12. Système modulaire de photobioréacteurs pour la culture d'algues selon l'une quelconque des revendications précédentes, **caractérisé en ce que** chaque photobioréacteur a une section tubulaire initiale (5) dans laquelle la culture d'algues est agencée équipée sur sa partie avant d'une série d'ouvertures réparties de manière égale (14), destinées à recevoir autant de conduits portant le produit qui est introduit dans le photobioréacteur.

13. Système modulaire de photobioréacteurs pour la culture d'algues selon l'une quelconque des revendications précédentes, **caractérisé en ce que** chaque lumière de type DEL (11) comprend deux types de DEL, rouge et bleue, avec deux spectres différents, et l'unité de commande comporte des moyens régulant l'activation de ces lumières (11) de deux types de DEL dans des sections tubulaires différentes (5) dans lesquelles la culture d'algues est agencée.

14. Système modulaire de photobioréacteurs pour la culture d'algues selon l'une quelconque des revendications précédentes, **caractérisé en ce que** chaque photobioréacteur incorpore une section tubulaire de sortie (5) dans laquelle la culture d'algues est agencée, qui est plus grande que les autres sections tubulaires (5) dans lesquelles la culture d'algues est agencée, qui constitue un réservoir de stockage et de séparation avec des refoulements supérieurs permettant de séparer l'écoulement d'algues huileuses, et de plusieurs refoulements inférieurs, ayant une aire d'écoulement plus grande que les refoulements supérieurs.

15. Système modulaire de photobioréacteurs pour la culture d'algues selon l'une quelconque des revendications précédentes, **caractérisé en ce que** chaque photobioréacteur comprend dans sa partie supérieure des vannes d'échappement réparties de manière égale pour faciliter la sortie d'excédent d'oxygène et/ou de pression.
